# DEMANDE DE BREVET EUROPEEN

(11) **EP 1 250 916 A1**
(43) Date de publication de la demande: **23.10.2002**
(21) Numéro de dépôt: 01810388.7
(22) Date de dépôt: 20.04.2001
(51) Int. Cl.: A61K 7/48

(54) **Agent émulsifiant sans PEG et son utilisation pour la préparation à température ambiante de compositions cosmétiques, pharmaceutiques et dermatologiques.**

(71) Demandeur: Dr. W. Kolb AG, 8908 Hedingen (CH)
(72) Inventeur: Meyer, Marie-Sophie, 8906 Bonstetten (CH); Nägeli, Ivo, 6300 Zug (CH)
(74) Mandataire: Isler & Pedrazzini AG

(57) **Abrégé**

La présente invention a pour objet un agent émulsifiant sans PEG comprenant d'une part un dérivé alkyl de sorbitan, et d'autre part un ester de polyglycérine. L'invention se rapporte aussi à l'utilisation de ce mélange pour la préparation à température ambiante de compositions cosmétiques, pharmaceutiques et dermatologiques.

## Description

La présente invention concerne un nouvel agent émulsifiant sans PEG (Poly Ethylène Glycol) pour la préparation à température ambiante de compositions cosmétiques, pharmaceutiques et dermatologiques.

Le mode classique d'émulsification consiste à chauffer jusqu'à 75-80°C la phase grasse et la phase aqueuse, l'une d'elles contenant l'émulsionnant. Cette phase de chauffage est indispensable si des composés solides tels que cires ou alcools gras sont présents dans le mélange, car ceux-ci doivent être sous forme liquide pour être émulsionnés. Puis les deux phases sont mélangées, selon le principe eau-dans-huile ou huile-dans-eau. L'émulsification est éventuellement complétée par une phase d'homogénéisation pour améliorer la distribution et la taille des gouttelettes de la phase dispersée. Les composés thermo-sensibles tels que parfum, huiles essentielles, vitamines, actifs divers (Dihydroxyacetone, Panthenol...) doivent être ajoutés à des températures inférieures à 40°C. Cette méthode nécessite un important apport d'énergie durant la phase de chauffage, chaleur par la suite éliminée lors d'une phase de refroidissement. Cette méthode, bien que largement utilisée, présente ainsi comme inconvénients majeurs une importante dépense d'énergie et de temps.

L'émulsification à température ambiante est, entre autre, un moyen de corriger les inconvénients précédemment cités, et permet ainsi de diminuer les coûts de fabrication. Cette méthode consiste à mélanger la phase grasse, la phase aqueuse et l'émulsionnant sous agitation mécanique afin de former une émulsion sans apport de chaleur, c'est-à-dire à une température ambiante (entre 20 et 30°C). Cela permet d'incorporer sans difficulté, sans risque et à n'importe quel moment du processus (avant ou après la phase d'émulsification) les composés thermo-sensibles tels que parfum, huiles essentielles, vitamines, actifs divers (Dihydroxyacetone, Panthenol...).

Trois critères limitent le champ d'application de ce principe. Premièrement, tous les composés à émulsionner doivent , à température ambiante, c'est-à-dire entre 20 à 30°C, soit se présenter sous forme liquide, soit être solubles dans l'une des phases. Deuxièmement, une température élevée (75-80°C) ne doit pas être un critère indispensable à la bonne réalisation de l'émulsion, par exemple lors de la présence dans le système de composés à haut point de fusion. Enfin, des problèmes de viscosité peuvent être rencontrés, un apport de consistance ne pouvant pas être réalisé par l'ajout de cires ou autres composés à haut point de fusion. Ce problème, qui a longtemps fortement limité le développement des émulsions à température ambiante, est maintenant résolu par l'introduction sur le marché de nouvelles substances apportant de la consistance et pouvant être travaillées à température ambiante.
En résumé, l'émulsification à température ambiante présente les avantages suivants par rapport à l'émulsification classique :
1. Economie d'énergie thermique (absence de phase de chauffage).
2. Economie de temps (absence de phase de chauffage).
3. Incorporation des composés thermo-sensibles à n'importe quel stade du processus.

L'émulsification à température ambiante est introduite en cosmétique dans les années 70 (Funke, *Seifen Öle Fette Wachse*, **1972,** *98(14),* 457-459 ; Schuster, *Parfümerie & Kosmetik,* **1977,** *58(12),* 353-365). Divers systèmes basés sur des émulsionnants avec PEG (DE 2511600 ; DE 3304897 ; WO 00/51550) ou avec émulsionnants cationiques (WO 01/08648) furent décrits dans des brevets. Cependant, les molécules avec PEG étant de plus en plus décrites comme induisant des intolérances cutanées, la recherche s'oriente vers le développement d'émulsionnants sans PEG.

La présente invention concerne de manière spécifique un nouvel agent émulsifiant sans PEG, ce qui signifie un système émulsifiant libre de traces de dioxane, d'oxyde d'éthylène et dérivés. Ce concept est exclusivement basé sur des produits d'origine naturelle (sorbitol, glycérine, acides gras). Un tel système présente, d'une part l'immense avantage d'une meilleure tolérance cutanée par rapport aux émulsionnants avec PEG, et d'autre part la possibilité de développer des émulsions dites « naturelles ». Les deux composants du système décrits par la présente invention, déjà connus individuellement en tant qu'émulsionnants, ont présenté la faculté inattendue à émulsionner divers types d'émollients à température ambiante, c'est-à-dire à une température comprise entre 0 et 50°C, particulièrement entre 10 et 40°C, et de manière optimale à 25°C. Ce système est composé d'un dérivé alkyl de sorbitan (a), dont la chaîne alkyle comprend 8 à 18 atomes de carbone, et 12 de manière optimale. Le second composant est un ester de polyglycérine (b), qui comprend 5 à 15 unités de glycérine, et 10 de manière optimale, et dont la chaîne alkyle comprend 8 à 18 atomes de carbone, et 12 de manière optimale.

Le système décrit par la présente invention comprend les deux composés (a) et (b) avec une proportion a : b comprise entre 99 : 1 et 20 : 80, plus particulièrement comprise entre 90 : 10 et 70 : 30, et de manière optimale 85 : 15. Cet agent émulsifiant est utilisé à une concentration de 0.001% à 50%, particulièrement entre 1 et 5%, et de manière optimale à 2%.

Les systèmes émulsifiants sont entre autre caractérisés par leur valeur HLB (Hydrophilic Lipophilic Balance). Le système HLB sert à représenter l'équilibre entre la partie hydrophile et la partie lipophile de la molécule émulsifiante. Un HLB de forte valeur décrit un émulsionnant plutôt hydrophile, donc plus soluble dans l'eau. Le HLB peut être calculé par diverses méthodes (méthode de Griffin 1949, méthode de Davies...). Lors de la préparation d'émulsions huile-dans-l'eau, le HLB du système émulsionnant est supérieur à 8, et de préférence supérieur ou égal à 10. Si l'on considère le dérivé alkyl de sorbitan avec une chaîne alkyle de 12 atomes de carbone, et l'ester de polyglycérine à 10 unités de glycérine, et une chaîne alkyle comprenant 12 atomes de carbone, ces deux composés ont respectivement un HLB d'environ 8.6 et 15. Ces deux agents émulsifiants sont alors mélangés dans des proportions variables, en fonction du HLB final désiré.

En résumé, ce système se laisse très bien travailler dans des conditions de température ambiante, c'est-à-dire à une température comprise entre 0 et 50°C, particulièrement entre 10 et 40°C, et de manière optimale à 25°C, avec divers types d'émollients et d'actifs, pour formuler des émulsions sans PEG, au toucher léger, faciles à appliquer, non collantes. Du fait de la large gamme d'actifs pouvant être émulsionnés par ce système, entre autre toute émulsion de type « skin care » et « sun care » peut être préparée :

### Incorporation d'émollients :

Cet agent émulsifiant permet d'émulsionner à température ambiante, c'est-à-dire à une température comprise entre 0 et 50°C, particulièrement entre 10 et 40°C, et de manière optimale à 25°C, divers types d'émollients, tels que, entre autres, des esters, des huiles d'origine végétale, certaines silicones... et des mélanges de celles-ci.

### Incorporation d'actifs :

Divers types d'actifs, thermo-sensibles ou non, peuvent être incorporés à température ambiante, c'est-à-dire à une température comprise entre 0 et 50°C, particulièrement entre 10 et 40°C, et de manière optimale à 25°C, dans des systèmes comprenant l'un des systèmes émulsionnants considérés par la présente invention.

Il s'agit, par exemple, de l'incorporation de filtres solaires anti-UVA et/ou anti-UVB. Ces filtres peuvent être de type chimique, tel que l'Octyl Methoxycinnamate, l'Octyl Salicylate ou l'Octocrylene, ou sous forme de solution aqueuse tel que le Phenylbenzimidazole Sulfonic Acid. Des filtres particulaires sous forme émulsionnée, tel que le Methylene Bis-Benzotriazolyl Tetramethylbutylphenol (Tinosorb M, Ciba) peuvent être également incorporés sans problème dans la phase aqueuse.
Divers types d'actifs liposolubles (entre autres, Bisabolol, Tocopherol, Tocopheryl Acetate...) ou hydrosolubles (entre autres, Allantoïn, Panthenol, Dihydroxyacetone...) peuvent être incorporés sans problème dans la phase aqueuse ou dans la phase grasse, selon leur solubilité, avant ou après l'émulsification. Du fait de l'absence de composés aminés dans le sysrème émulsifiant considéré par l'invention, la Dihydroxyacetone peut être incorporée aux doses recommandées pour une coloration suffisante de la peau (5 à 8%) sans problème d'incompatibilité ou de stabilité.

### Exemples :

Les exemples suivants, non limitatifs, donnent une idée des applications du système émulsionnant considéré :

### Exemple 1 : Préparation d'un agent émulsifiant selon l'invention :

Préparation de l'agent émulsionnant composé de Sorbitan Laurate et Polyglycerine-10 Laurate :
Dans un réacteur de 1kg muni d'une double enveloppe, verser 425g de Sorbitan Laurate et 75g de Polyglycerine-10 Laurate. Agiter et chauffer à 65°C. Lorsque la température de 65°C est atteinte, continuer à agiter modérément pendant 1 heure. Refroidir à 40°C. Vider le réacteur.

Les exemples ci-après décrivent des formulations dans lesquelles le système émulsionnant proposé dans l'invention a été testé. Chaque formulation a subi les tests de stabilité suivants :
- Centrifugation : 10 min ; TA ; 5000 tr/min ; r = 7.61 cm.
- Stockage à TA (température ambiante : env. 25°C) pendant 12 semaines.
- Stockage à 4°C pendant 12 semaines.
- Stockage à 45°C pendant 12 semaines.
- Stockage à des cycles de température (TA / -12°C / TA / 45°C) pendant 6 cycles.

### Exemple 2 : Préparation d'une émulsion huile-dans-eau :

L'agent émulsifiant décrit dans cette invention permet la préparation d'émulsions huile-dans-eau à température ambiante, c'est-à-dire à une température comprise entre 0 et 50°C, particulièrement entre 10 et 40°C, et de manière optimale à 25°C. La concentration d'utilisation de cet agent émulsifiant se situe entre 2% et 5%.

Quatre types de phases grasses ont été choisies et émulsionnées avec l'agent émulsifiant, afin de définir le domaine d'émulsification de ce système. Le tableau 1 résume ces essais.

**Tableau 1**

| **Phases grasses** | **% Wt.** | **Stabilité (semaines)** | | | **Stabilité (cycles)** |
|---|---|---|---|---|---|
| **noms I.N.C.I.** | | ***TA***^{***6***} | ***4°C*** | ***45°C*** | ***Cycle***^{***7***} |
| Octyl Caprylate¹ | 6% | > 8 | > 8 | > 8 | > 6 |
| Tridecyl Stearate² | 9% | | | | |
| Octyl Stearate³ | 7% | > 8 | > 8 | > 8 | > 6 |
| C₁₂₋₁₅ Alkyl Benzoate⁴ | 8% | | | | |
| Persea gratissima⁵ | 7% | > 8 | > 8 | > 8 | > 6 |
| Isopropyl Palmitate | 8% | | | | |
| C₁₂₋₁₅ Alkyl Benzoate⁴ | 14% | > 8 | > 8 | > 8 | > 6 |
| Dimethicone | 1% | | | | |

| | | | | | |
|---|---|---|---|---|---|
| ¹ Sympatens™-EC (Kolb) | | | | | |
| ² Sympatens™-ITS (Kolb) | | | | | |
| ³ Sympatens™-ES (Kolb) | | | | | |
| ⁴ Sympatens™-LBZ (Kolb) | | | | | |
| ⁵ Huile d'avocat | | | | | |
| ⁶ Température ambiante (env. 25°C) | | | | | |
| ⁷ TA / -12°C / TA / 45°C. | | | | | |

Le tableau 2 montre un exemple d'émulsion huile-dans-eau selon l'invention.

**Tableau 2**

| **Phases** | **Matières Premières - noms I .N.C.I.** | **% Wt.** |
|---|---|---|
| A | *Emulsionnant*^{*1*} | 2.00 |
| | *Phase grasse*^{*2*} | 15.00 |
| | | |
| B | Aqua | q.s.p. 100 |
| | Glycerin | 3.00 |
| | Propylene Glycol | 1.00 |
| | Ultrez 10 | 0.10 à 0.12 |
| | | |
| C | Triethanolamine | 0.10 à 0.12 |
| | Tocopherol | 0.05 |
| | Preservative | q.s. |
| | Parfum | q.s. |

| | | |
|---|---|---|
| ¹ Produit selon l'exemple 1 | | |
| ² Emollients selon l'exemple 2, tableau 1 | | |

Mode opératoire :
- Agitation modérée à température ambiante (environ 25°C) des phases A et B séparément, jusqu'à obtention de phases homogènes.
- Ajout de la phase A dans la phase B (émulsification par voie directe) sous agitation plus vive.
- Homogénéisation pendant 5 minutes à environ 500 trs/min.
- Ajout de la phase C.

### Exemple 3 : Préparation d'une émulsion après-solaire :

Le tableau 3 montre un exemple d'émulsion huile-dans-eau selon l'invention, comprenant des actifs utilisés pour le soin de la peau ayant été exposée au soleil.

**Tableau 3**

| **Phases** | **Matières Premières - noms I .N.C.I.** | **% Wt.** |
|---|---|---|
| A | *Emulsionnant*^{*1*} | 2.00 |
| | C₁₂₋₁₅ Alkyl Benzoate² | 7.00 |
| | Isotridecyl Stearate³ | 8.00 |
| | | |
| B | Aqua | q.s.p. 100 |
| | Glycerin | 3.00 |
| | Propylene Glycol | 1.00 |
| | Ultrez 10 | 0.12 |
| | | |
| C | Triethanolamine | 0.12 |
| | Panthenol | 0.50 |
| | Bisabolol | 0.30 |
| | Tocopheryl Acetate | 0.20 |
| | Preservative | q.s. |
| | Parfum | q.s. |

| | | |
|---|---|---|
| ¹ Produit selon l'exemple 1 | | |
| ² Sympatens™-LBZ (Kolb) | | |
| ³ Sympatens™-ITS (Kolb) | | |

Mode opératoire :
- Agitation modérée à température ambiante (environ 25°C) des phases A et B séparément, jusqu'à obtention de phases homogènes.
- Ajout de la phase A dans la phase B (émulsification par voie directe) sous agitation plus vive.
- Homogénéisation pendant 5 minutes à environ 500 trs/min.
- Ajout de la phase C.

Les actifs employés dans cet exemple ne sont pas exhaustifs. D'autres, tels que Allantoïne ou Aloe vera par exemple, peuvent également être employés pour la préparation d'une émulsion après-solaire avec émulsification à température ambiante.

### Exemple 4 : Préparation d'une émulsion solaire :

Le tableau 4 montre un exemple d'émulsion huile-dans-eau selon l'invention, comprenant des filtres solaires anti-UVA et anti-UVB.

**Tableau 4**

| **Phases** | **Matières Premières - noms I .N.C.I.** | **% Wt.** |
|---|---|---|
| *A* | *Emulsionnant*^{*1*} | 2.00 |
| | C₁₂₋₁₅ Alkyl Benzoate² | 8.00 |
| | Octyl Stearate³ | 7.00 |
| | Octyl Methoxycinnamate | 5.00 |
| | | |
| B | Aqua | q.s.p. 100 |
| | Glycerin | 3.00 |
| | Methylene Bis-Benzotriazolyl Tetramethylbutylphenol (and) ⁴ | 8.00 |
| | Ultrez 10 | 0.15 |
| | Sodium Hydroxyde 10% | 0.30 |
| | | |
| C | Tocopheryl Acetate | 0.30 |
| | Preservative | q.s. |
| | Parfum | q.s. |

| | | |
|---|---|---|
| ¹ Produit selon l'exemple **1** | | |
| ² Sympatens™-LBZ (Kolb) | | |
| ³ Sympatens™-ES (Kolb) | | |
| ⁴ Decyl Glucoside (and) Xanthan Gum (and) Propylene Glycol (and) Aqua (Tinosorb M, Ciba) | | |

Mode opératoire :
- Agitation modérée à température ambiante (environ 25°C) des phases A et B séparément, jusqu'à obtention de phases homogènes.
- Ajout de la phase A dans la phase B (émulsification par voie directe) sous agitation plus vive.
- Homogénéisation pendant 5 minutes à environ 500 trs/min.
- Ajout de la phase C.

Les actifs solaires pouvant être employés dans cet exemple ne sont pas listés de manière exhaustive. D'autres filtres chimiques liquides, tels que l'Octyl Methoxycinnamate, l'Octyl Salicylate, l'Octocrylene, entre autres, ou sous forme de solution aqueuse tel que le Phenylbenzimidazole Sulfonic Acid par exemple, peuvent également être utilisés pour la préparation d'une émulsion solaire avec émulsification à température ambiante. L'emploi de filtres chimiques solides n'est pas exclu, mais soumis à la condition que les filtres en question soient solubles dans l'une des phases.

### Exemple 5 : Préparation d'une émulsion auto-bronzante :

Le tableau 5 montre un exemple d'émulsion huile-dans-eau selon l'invention, comprenant 5% d'actif auto-bronzant (Dihydroxyacetone) :

**Tableau 5**

| **Phases** | **Matières Premières - noms I .N.C.I.** | **% Wt.** |
|---|---|---|
| A | *Emulsionnant*^{*1*} | 2.00 |
| | C₁₂₋₁₅ Alkyl Benzoate² | 8.00 |
| | Octyl Stearate³ | 7.00 |
| | | |
| B | Aqua | q.s.p. 100 |
| | Glycerin | 3.00 |
| | Propylene Glycol | 1.00 |
| | Xanthan Gum | 1.00 |
| | | |
| C | Dihydroxyacetone | 5.00 |
| | Preservative | q.s. |
| | Parfum | q.s. |

| | | |
|---|---|---|
| ¹ Produit selon l'exemple 1 | | |
| ² Sympatens™-LBZ (Kolb) | | |
| ³ Sympatens™-ES (Kolb) | | |

Mode opératoire :
- Agitation modérée à température ambiante (environ 25°C) des phases A et B séparément, jusqu'à obtention de phases homogènes.
- Ajout de la phase A dans la phase B (émulsification par voie directe) sous agitation plus vive.
- Homogénéisation pendant 5 minutes à environ 500 trs/min.
- Ajout de la phase C.

## Revendications

1. Un agent émulsifiant sans PEG comprenant d'une part un dérivé alkyl de sorbitan (composé a), et d'autre part un ester de polyglycérine (composé b), utilisé comme émulsionnant à température ambiante.

2. Un agent émulsifiant selon la revendication 1, constitué des composés a et b, avec une proportion a : b comprise entre 99: 1 et 20: 80, plus particulièrement comprise entre 90 : 10 et 70 : 30, et de manière optimale 85 : 15.

3. Un agent émulsifiant selon les revendications 1 à 2, **caractérisé en ce que** la chaîne alkyle du dérivé alkyl de sorbitan comprend 8 à 18 atomes de carbone, et 12 de manière optimale.

4. Un agent émulsifiant selon les revendications 1 à 2, **caractérisé en ce que** l'ester de polyglycérine comprend 5 à 15 unités de glycérine, et 10 de manière optimale.

5. Un agent émulsifiant selon les revendications 1 à 2, **caractérisé en ce que** la chaîne alkyle de l'ester de polyglycérine comprend 8 à 18 atomes de carbone, et 12 de manière optimale.

6. Utilisation d'un agent émulsifiant selon les revendications 1 à 5 pour la préparation de compositions cosmétiques, pharmaceutiques et dermatologiques à une température comprise entre 0 et 50°C, particulièrement entre 10 et 40°C, et de manière optimale à 25°C.

7. Préparation de compositions cosmétiques, pharmaceutiques et dermatologiques selon les revendications 1 à 6, **caractérisées en ce qu'**elles comprennent 0.001% à 50%, particulièrement 1 à 5%, et de manière optimale 2% du produit considéré par la présente invention.

8. Compositions selon les revendications 1 à 7 se présentant sous la forme d'une solution, d'une émulsion eau-dans-huile (E/H) ou huile-dans-eau (H/E), d'une émulsion multiple (E/H/E ou H/E/H), d'une préparation anhydre, d'un gel ou d'un aérosol.
